# EUROPÄISCHE PATENTANMELDUNG

(11) **EP 3 072 589 A1**
(43) Veröffentlichungstag der Anmeldung: **28.09.2016**
(21) Anmeldenummer: 15161099.5
(22) Anmeldetag: 26.03.2015
(51) Int. Cl.: B01J 23/656, B01J 37/04, B01J 21/06, B01J 35/00, B01J 35/10, B01J 37/02, C10G 2/00

(54) **Katalysator und Verfahren zur selektiven Methanisierung von Kohlenmonoxid**

(71) Anmelder: BASF SE, 67056 Ludwigshafen am Rhein (DE)
(72) Erfinder: Milanov, Adrian, 68199 Mannheim (DE); Schwab, Ekkehard, 67434 Neustadt (DE); Hoffmann, Mike, 67657 Kaiserslautern (DE); Kotrel, Stefan, 69469 Weinheim (DE); Altwasser, Stefan, 70173 Stuttgart (DE)
(74) Vertreter: BASF IP Association

(57) **Zusammenfassung**

Die vorliegende Erfindung betrifft eine katalytisch aktive Zusammensetzung für die selektive Methanisierung von Kohlenmonoxid in wasserstoff- und kohlendioxidhaltigen Reformatströmen, enthaltend als Aktivkomponente wenigstens ein Element, ausgewählt aus der Gruppe, bestehend aus Ruthenium, Rhodium, Nickel und Kobalt und als Dotierungsmittel Rhenium auf einem Trägermaterial. Der erfindungsgemäße Katalysator wird bevorzugt zur Durchführung von Methanisierungsreaktionen in einem Temperaturbereich von 100 bis 300°C für den Einsatz bei der Wasserstofferzeugung für Brennstoffzellenanwendungen eingesetzt.

## Beschreibung

Die Erfindung betrifft eine katalytische Zusammensetzung und ein Verfahren zur selektiven Methanisierung von Kohlenmonoxid in wasserstoff- und kohlendioxidhaltigen Strömen, insbesondere für die Verwendung in Brennstoffzellensystemen.

Niedertemperatur-PEM-Brennstoffzellen (PEM = Polymer Electrolyte Membrane) können nur mit Wasserstoff oder wasserstoffreichen Gasen einer definierten Qualität betrieben werden. Dabei ist insbesondere die Kohlenmonoxid (CO)-Konzentration eine kritische Größe. Sie hängt vom eingesetzten Energieträger und vom verwendeten Reformierungsverfahren ab. Die Entfernung von höheren CO-Konzentrationen ist mit der Wassergas-Shift-Reaktion unter weiterer Bildung von Wasserstoff möglich.

CO + H₂O ↔ CO₂ + H₂ ΔH = -44 kJ/mol

Da es sich um eine Gleichgewichtsreaktion handelt, verbleibt in Abhängigkeit von Verfahrensauslegung und Temperatur eine Restkonzentration an CO im Gasstrom in der Regel im Bereich von 0,25 bis 1,5 Vol.-%. Bei Verwendung von Katalysatoren mit hohem Kupfergehalt kann beispielsweise eine CO-Entfernung bis auf 2.500 ppm erreicht werden. Der CO-Gehalt im wasserstoffreichen Gas muss allerdings noch weiter reduziert werden, um eine Vergiftung des Anodenkatalysators zu vermeiden; Richtwerte liegen hier zwischen maximal 10 und 50 ppm.

Die Reduzierung des enthaltenen CO aus dem Gasstrom bis unter die erforderlichen Grenzwerte erfolgt üblicherweise in einer Feinreinigungsstufe. Dabei ist heute die selektive Oxidation die gängige CO-Entfernungsmethode. Die selektive Oxidation weist einen hohen Entwicklungsstand auf, besitzt aber neben dem Nachteil einer nur mäßigen Selektivität die Notwendigkeit einer exakt dosierten Luftzufuhr, woraus ein hoher mess- und regelungstechnischer Aufwand resultiert. Wird das notwendige Verhältnis von Sauerstoff zu CO nicht exakt eingehalten, kann dies zu hohen Verlusten an Wasserstoff führen. Weiterhin erfordert das schmale Temperaturfenster von in der Regel maximal 20°C ein aufwendiges Thermomanagement des Reaktors. Hinzu kommt über die Zumischung des Oxidationsmittels Sauerstoff zum Gas eine sicherheitstechnische Problematik. Die Entfernung des CO durch Reaktion mit H₂ (selektive Methanisierung von CO in Anwesenheit von CO₂) hat gegenüber der selektiven CO-Oxidation durch ihre verfahrenstechnisch anspruchslose Realisierung erhebliche Vorteile.

Die CO-Methanisierung (Hydrierung von Kohlenstoffmonoxid zu Methan) erfolgt nach der Reaktionsgleichung:

CO + 3H₂ → CH₄ + H₂O ΔH = -206,2 kJ/mol

Als Konkurrenzreaktion läuft die Umwandlung von CO₂ zu Methan ab:

CO₂ + 4H₂ → CH₄ + 2H₂O ΔH = -164,9 kJ/mol

Die besondere Herausforderung für die selektive CO-Methanisierung liegt darin, dass bevorzugt CO hydriert werden soll und nicht CO₂, da dies weiteren Wasserstoff verbrauchen würde. Die CO-Konzentration liegt im Reformat bei ca. 2500 ppm bis 15.000 ppm, während der CO₂-Gehalt mit ca. 15 bis 25 Vol.-% eine Größenordnung über dem CO-Gehalt liegt. Dementsprechend ist ein CO-selektiver Katalysator für die Realisierung der niedrigen CO-Konzentrationen, wie sie für z. B. PEM-Brennstoffzellen verlangt werden, unabdingbar.

Die selektive Methanisierung von CO ist seit langem bekannt. Zunächst wurde CO am Nickelkatalysator methanisiert, wobei CO₂ jedoch zuvor ausgewaschen werden musste. 1968 wurde ein Rutheniumkatalysator zur selektiven CO-Methanisierung von Baker et al. beansprucht (US 3,615,164), wobei dort ein Ruthenium- oder Rhodiumkatalysator auf einem Aluminiumoxid-Trägermaterial verwendet wird. Ebenso ist in Chemical Abstracts, Band 74, 1971, Nr. 35106u, die selektive Methanisierung von CO in einem Gasgemisch, das Wasserstoff, Kohlendioxid und Kohlenmonoxid enthält, bei Temperaturen im Bereich zwischen 125 und 300°C unter Verwendung rutheniumhaltiger Katalysatoren beschrieben. In US 3,663,162 von 1972 wird ein Raney-Nickel-Katalysator für diese Reaktion beansprucht.

In EP-A-1174486 wird eine Methanisierungsstufe mit einer Einheit zur selektiven Oxidation mit dem Ziel eines geringeren Sauerstoffverbrauches und einer geringeren CO₂-Methanisierungsrate kombiniert. Der für die Methanisierung eingesetzte Katalysator enthält Ru, Pt, Rh, Pd oder Ni auf einem Aluminiumoxidträger.

In WO 98/13294 werden zwei Methanisierungsstufen unterschiedlicher Temperaturniveaus zusammengeschaltet. Vorteil soll hier sein, dass bei der Hochtemperaturstufe noch kein oder weniger CO₂ methanisiert, aber schon ein großer Teil des Kohlenmonoxids abgebaut wird. In der sich anschließenden Tieftemperaturmethanisierung erfolgt die Restentfernung von CO. Verwendet wird ein Edelmetallkatalysator, insbesondere Rh, auf einem Aluminiumträger.

WO 97/43207 beschreibt die Kombination einer ersten Stufe zur selektiven Oxidation mit einer nachfolgenden Methanisierungsstufe mit Rhodium als Aktivkomponente. Mit dieser Kombination sollen sich beide Prozesse unter optimalen Bedingungen betreiben lassen.

Weitere neuere Anmeldungen, wie beispielsweise EP-A-1246286, in der als letzte Prozessstufe einer Gasreinigung ein Methanisierungsreaktor einer Einheit zur selektiven Oxidation mit der Begründung des einfacheren Aufbaus und der besseren Handhabung nachgeschaltet wird, verwenden herkömmliche Katalysatoren, überwiegend auf Ruthenium- oder Nickelbasis.

JP-A-2002/068707 behandelt Methanisierungskatalysatoren, die auf einem feuerfesten anorganischen Oxid, ausgewählt aus Oxiden von Aluminium, Titan, Silizium oder Zirkonium, aufgebracht sind.

EP-A-1707261 beschreibt ein Verfahren zur selektiven Oxidation von CO mit einem Katalysator, der Ruthenium auf einem Träger aus Mischmetalloxiden enthält, dotiert mit Lanthaniden.

US 7,560,496 beschreibt ein Verfahren zur selektiven Methanisierung von CO in Gegenwart von CO₂ unter Verwendung eines Katalysators, der Ruthenium, Rhodium, Nickel und/oder Kobalt als aktive Komponente und mindestens einen weiteren Dotierstoff ausgewählt aus der Gruppe, bestehend aus Eisen, Niob, Mangan, Molybdän und Zirkonium auf einem Trägermaterial auf Kohlebasis enthält.

WO 2008/101875 betrifft eine katalytisch aktive Zusammensetzung für die selektive Methanisierung von Kohlenmonoxid in wasserstoff- und kohlendioxidhaltigen Strömen, die dadurch gekennzeichnet ist, dass sie als Aktivkomponente Ruthenium und als Trägermaterial ein Lanthan-Cer-Zirkonoxid enthält.

US-A-2005/0096212 beschreibt die selektive Methanisierung an einem Katalysator aus Ru, Rh, Ni oder Kombinationen auf β-Zeolith, Mordenit und Faujasit. Zwar werden so die gewünschten CO-Konzentrationen unterhalb von 100 ppm erreicht, doch sinkt die Selektivität bei Temperaturen über 190°C, bei denen der Katalysator seine Aktivität entfaltet, deutlich unter 50%. Da die Hydrierung von CO₂ pro Mol 3/2 mal so viel Wasserstoff vernichtet wie die Hydrierung von CO, ist die Forderung nach möglichst hoher Selektivität sehr wichtig. Außerdem wird eine vernünftige katalytische Aktivität nur über das sehr kleine Temperaturfenster zwischen 170°C und 180°C erreicht.

Die Verfahren des Standes der Technik gestatten es nicht, eine ausreichende Senkung des CO-Gehaltes unter Schonung des CO₂-Gehaltes zu gewährleisten. Die bisher entwickelten Katalysatoren arbeiten entweder nicht selektiv genug oder wirken nur in einem sehr schmalen Temperaturbereich. Vor allem der sehr schmale Temperaturbereich macht eine technische Realisierung des Konzeptes "Selektive Methanisierung" sehr schwierig. Denn sobald die Selektivität sinkt, kommt es zur Erwärmung des Reaktors, was zu einer weiteren Methanisierung von CO₂ und damit zum thermischen "Durchgehen" der Prozesseinheit führt. Durch die Exothermie der Reaktion kommt es also zu Hot-Spots. Aus diesem Grund muss ein breites Temperaturfenster fahrbar sein. Ebenso ein Problem ist die adiabate Temperaturerhöhung in Monolithen, wenn diese als Katalysatorformkörper eingesetzt werden, was in der Praxis oftmals der Fall ist.

Insbesondere für Brennstoffzellenanwendungen stellt der geforderte CO-Maximalgehalt im eingespeisten wasserstoffreichen Gas und die notwendige hohe Selektivität (Methanisierung von CO, aber nicht von CO₂) über ein breites Temperaturfenster noch ein großes Entwicklungspotential für geeignete deaktivierungsresistente Katalysatoren dar.

Die Aufgabe der Erfindung bestand damit in der Bereitstellung eines Katalysators für die selektive CO-Methanisierung, der seine Selektivität und Aktivität in einem breiten Temperaturbereich erhält.

Die Aufgabe wurde erfindungsgemäß dadurch gelöst, dass für die selektive Methanisierung von Kohlenmonoxid in wasserstoff- und kohlendioxidhaltigen Reformatströmen eine katalytisch aktive Zusammensetzung eingesetzt wird, die Ruthenium, Rhodium, Nickel, oder Cobalt oder Mischungen davon als Aktivkomponente und Rhenium als Dotierelement auf einem geeigneten Trägermaterial enthält.

Es wurde überraschend gefunden, dass ein Katalysator, der Ruthenium, Rhodium, Nickel oder Kobalt oder Mischungen davon als Aktivkomponente und Rhenium als Dotierelement auf einem geeigneten Trägermaterial enthält, in der Lage ist, die Methanisierung von CO in einem breiten Temperaturbereich von etwa 100 bis 300°C in einer nahezu konstanten Selektivität über eine lange Zeitspanne zu gewährleisten. Herkömmliche Katalysatoren zeigen mit zunehmender Temperatur und längeren Laufzeiten einen deutlichen Selektivitätsabfall. Durch Anwendung des erfindungsgemäßen Katalysators ist ein deutlich geringerer Regelaufwand erforderlich, da das Temperaturfenster bei der Methanisierung des CO weniger exakt eingehalten werden muss. Darüber hinaus kann ein auch bei hohen Temperaturen gut arbeitender Katalysator direkt der Vorreinigungsstufe (TTK - Tieftemperaturkonvertierung), die bei etwa 220 bis 280°C betrieben wird, nachgeschaltet werden.

Gegenstand der Erfindung ist damit eine katalytisch aktive Zusammensetzung für die selektive Methanisierung von Kohlenmonoxid in wasserstoff- und kohlendioxidhaltigen Reformatströmen, enthaltend als Aktivkomponente wenigstens ein Element ausgewählt aus der Gruppe bestehend aus Ruthenium, Rhodium, Nickel und Kobalt und als Dotierungsmittel Rhenium auf einem Trägermaterial.

Die Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in den jeweils angegebenen Kombinationen, sondern auch in anderen Kombinationen verwendbar sind, ohne den Rahmen der Erfindung zu verlassen.

Die katalytisch aktive Zusammensetzung enthält als Aktivkomponente wenigstens ein Element, ausgewählt aus der Gruppe bestehend aus Ruthenium, Rhodium, Nickel und Kobalt, vorzugsweise Ruthenium. Die Aktivkomponente liegt dabei im Katalysator vorzugsweise als Oxid vor. Die eigentliche Aktivmasse wird dann durch Aktivierung mit Wasserstoff in situ erzeugt.

Eine ex-situ Vorreduktion mit z.B. Wasserstoff, Formiergas oder einem anderen geeigneten Reduktionsmittel ist ebenfalls möglich. In diesem Fall liegt die Aktivkomponente des Katalysators in metallischer Form vor. Der Katalysator kann dann sowohl in dieser Form als auch nach einer anschließenden Oberflächen-Passivierung der metallischen Komponente im Reaktor eingebaut werden.

Als Trägermaterial sind erfindungsgemäß alle üblicherweise in der Katalysatorchemie für diese Zwecke einsetzbaren Materialien geeignet, die eine ausreichend hohe BET-Oberfläche und eine entsprechende Porosität (Porenvolumen) aufweisen. Beispielhaft zu nennen sind Trägermaterialien ausgewählt aus der Gruppe, bestehend aus Al₂O₃, ZrO₂, TiO₂, SiC, ZnO, Oxiden der Gruppe IIA Metalle, Oxiden der Gruppen IIIB, IVB, VB, VIB Übergangsmetalle, Oxiden von Metallen der Seltenen Erden, Aluminosilicate, Zeolite, MOFs (Metal Organic Framework) sowie deren Mischungen.

Als bevorzugt verwendete Träger sind erfindungsgemäß solche Materialien, ausgewählt aus der Gruppe, bestehend aus Al₂O₃, ZrO₂, TiO₂, und Oxiden von Metallen der Seltenen Erden zu nennen.

Ein erfindungsgemäß besonders bevorzugtes Trägermaterial ist ein Lanthan-Cer-Zirkonoxid (LaCeZr-Oxid) mit einem Lanthanoxid-Gehalt von 0,1 bis 15 Gew.-%, bevorzugt von 1 bis 10 Gew.-% und besonders bevorzugt von 3 bis 7 Gew.-% eingesetzt. Der Ceroxid-Gehalt beträgt 0,1 bis 20 Gew.-%, bevorzugt 1 bis 17 Gew.-% und besonders bevorzugt von 10 bis 16 Gew.-%, jeweils bezogen auf das Gewicht des gesamten Trägermaterials.

Der Zirkonoxid-Gehalt des Trägermaterials beträgt vorteilhafterweise 30 bis 99,8 Gew.-%. In bevorzugten Ausführungen liegt er bei einem Gehalt, der die Gewichtsanteile des Lanthanoxids und Ceroxids und ggf. weiterer Bestandteile, wie nachfolgend beschrieben, zu jeweils 100 Gew.-% ergänzt.

In einer bevorzugten Ausführungsform ist der erfindungsgemäß verwendete Träger dadurch gekennzeichnet, dass dessen physikochemischen Eigenschaften wie BET-Oberfläche, Porenvolumen und Seitendruckfestigkeit bevorzugte Werte aufweisen. Die BET-Oberfläche der für die erfindungsgemäßen Katalysatoren verwendeten Trägermaterialen liegt bei mindestens 10 m²/g, vorteilhafterweise bei mindestens 20 m²/g, bevorzugt bei mindestens 40 m²/g, besonders bevorzugt bei mindestens 60m²/g und ganz besonders bevorzugt bei mindestens 80m²/g. Die Bestimmung der BET Oberfläche erfolgt nach einer Methode gemäß der DIN 66131.

Das Porenvolumen des Trägermaterials liegt vorteilhafterweise im Bereich von 0,05 bis 1,5 cm³/g, bevorzugt im Bereich von 0,1 bis 1,0 cm³/g, besonders bevorzugt im Bereich von 0,15 bis 0,9 cm³/g, ganz besonders bevorzugt im Bereich von 0,17 bis 0,7 cm³/g, insbesondere im Bereich von 0,2 bis 0,6 cm³/g. Die Bestimmung des Porenvolumens erfolgt nach der Methode der Quecksilberporosimetrie nach DIN 66133.

Für Trägermaterialien in Form von Formkörpern (wie z.B. Tabletten, Extrudate, kugelförmige Partikel, etc.) liegt deren Druckfestigkeit vorteilhafterweise bei mindestens 0,2 kgf, bevorzugt bei mindestens 0,5 kgf, besonders bevorzugt bei mindestens 1,0 kgf, ganz besonders bevorzugt bei mindestens 1,5 kgf, insbesondere bei mindestens 2,0 kgf. Die Seitendruckfestigkeit ist ein Maß für die Stabilität eines Materials bei Ausübung von Druck auf seine Seitenflächen. Das Material wird dazu zwischen zwei Stempeln eingespannt (Vorkraft 0,5 N), die sich dann mit 1,6 mm/min Prüfgeschwindigkeit aufeinander zu bewegen und das Material zerdrücken. Die Kraft, die zum Zerdrücken des Materials benötigt wird, wird aufgezeichnet. Daten ergeben sich über eine statistische Auswertung von wenigstens 20 Formkörpern.

Das erfindungsgemäß verwendete Trägermaterial kann neben den genannten Komponenten weitere üblicherweise in der Katalysatorchemie für diese Zwecke einsetzbare Materialien enthalten, wie beispielsweise Aluminiumoxid. Geeignet sind solche Bindermaterialien, die eine ausreichend hohe BET-Oberfläche aufweisen. Vorteilhafterweise sollte die BET-Oberfläche dieser zusätzlich eingesetzten Bindermaterialien mindestens 120 m²/g betragen. Der Gehalt an diesen Bindermaterialien sollte dabei 70 Gew.-%, bevorzugt 50 Gew.-%, besonders bevorzugt 30 Gew.-%, und ganz besonders bevorzugt 20 Gew.-%, jeweils bezogen auf das Gewicht des gesamten Trägermaterials, nicht überschreiten.

Die Beladung des Trägermaterials mit wenigstens einer der oben genannten erfindungsgemäßen Aktivkomponenten beträgt 0,1 bis 20 Gew.-%, bevorzugt 0,2 bis 10 Gew.-%, besonders bevorzugt 0,3 bis 5 Gew.-%, ganz besonders bevorzugt 0,4 bis 4,5 Gew.-%, insbesondere 0,5 bis 3 Gew.-%. Weitere vorteilhafte Mengenbereiche sind beispielsweise 0,1 bis 10 Gew.-%, 0,5 bis 5 Gew.-% sowie 0,7 bis 4 und 1 bis 3 Gew.-%. Die Angaben sind jeweils bezogen auf das Gesamtgewicht der katalytisch aktiven Zusammensetzung.

Die Beladung des Trägermaterials mit Rhenium als Dotierelement beträgt 0,01 bis 20 Gew.-%, bevorzugt 0,05 bis 10 Gew.-%, besonders bevorzugt 0,07 bis 5 Gew.-%, ganz besonders bevorzugt 0,08 bis 4 Gew.-%, insbesondere 0,1 bis 3 Gew.-%. Die Angaben sind jeweils bezogen auf das Gesamtgewicht der katalytisch aktiven Zusammensetzung.

Eine bevorzugte Zusammensetzung des erfindungsgemäßen katalytisch aktiven Systems enthält 0,01 bis 20 Gew.-%, bevorzugt 0,07 bis 5 Gew.-%, besonders bevorzugt 0,08 bis 4 Gew.-% Rhenium und 0,1 bis 20 Gew.-%, bevorzugt 0,5 bis 5 Gew.-%, besonders bevorzugt 1 bis 3 Gew.-% Ruthenium, bezogen auf das Gesamtgewicht der katalytisch aktiven Zusammensetzung auf einem Träger ausgewählt aus der Gruppe, bestehend aus Al₂O₃, ZrO₂, TiO₂ und Metalloxiden der Seltenen Erden, bevorzugt auf einem Träger aus ZrO₂.

Eine weitere bevorzugte Zusammensetzung des erfindungsgemäßen katalytisch aktiven Systems enthält 0,01 bis 20 Gew.-%, bevorzugt 0,07 bis 5 Gew.-%, besonders bevorzugt 0,08 bis 4 Gew.-% Rhenium und 0,1 bis 20 Gew.-%, bevorzugt 0,2 bis 10 Gew.-%, besonders bevorzugt 0,3 bis 5 Gew.-% Ruthenium, bezogen auf das Gesamtgewicht der katalytisch aktiven Zusammensetzung auf einem Träger ausgewählt aus der Gruppe, bestehend aus Zeolit A, ß-Zeolit, Mordenit, Faujasit, ZSM-5 und MOF.

Eine weitere bevorzugte Zusammensetzung des katalytisch aktiven Systems enthält auf einem Lanthan-Cer-Zirkonoxid-Träger mit einem Lanthanoxid-Gehalt von 0,1 bis 15 Gew.-% und einem Ceroxid-Gehalt von 0,1 bis 20 Gew.-%, jeweils bezogen auf das Gewicht des gesamten Trägermaterials, 0,01 bis 20 Gew.-% Rhenium und 0,1 bis 20 Gew.-% Ruthenium, bezogen auf das Gesamtgewicht der katalytisch aktiven Zusammensetzung.

Eine weitere bevorzugte Zusammensetzung des katalytisch aktiven Systems enthält auf einem Lanthan-Cer-Zirkonoxid-Träger mit einem Lanthanoxid-Gehalt von 0,1 bis 15 Gew.-% und einem Ceroxid-Gehalt von 0,1 bis 20 Gew.-%, jeweils bezogen auf das Gewicht des gesamten Trägermaterials, 0,05 bis 10 Gew.-% Rhenium und 0,5 bis 5 Gew.-% Ruthenium, bezogen auf das Gesamtgewicht der katalytisch aktiven Zusammensetzung.

Eine weitere bevorzugte Zusammensetzung des katalytisch aktiven Systems enthält auf einem Lanthan-Cer-Zirkonoxid-Träger mit einem Lanthanoxid-Gehalt von 0,1 bis 10 Gew.-% und einem Ceroxid-Gehalt von 0,1 bis 17 Gew.-%, jeweils bezogen auf das Gewicht des gesamten Trägermaterials, 0,07 bis 5 Gew.-% Rhenium und 0,7 bis 4 Gew.-% Ruthenium, bezogen auf das Gesamtgewicht der katalytisch aktiven Zusammensetzung.

Eine besonders bevorzugte Zusammensetzung des katalytisch aktiven Systems enthält auf einem Lanthan-Cer-Zirkonoxid-Träger mit einem Lanthanoxid-Gehalt von 3 bis 7 Gew.-% und einem Ceroxid-Gehalt von 10 bis 16 Gew.-%, jeweils bezogen auf das Gewicht des gesamten Trägermaterials, 0,08 bis 4 Gew.-% Rhenium und 1 bis 3 Gew.-% Ruthenium, bezogen auf das Gesamtgewicht der katalytisch aktiven Zusammensetzung.

In einer bevorzugten Ausführungsform ist der erfindungsgemäße Katalysator dadurch gekennzeichnet, dass dessen physikochemischen Eigenschaften wie die Phasenzusammensetzung nach XRD, BET-Oberfläche, Porenvolumen und Seitendruckfestigkeit bevorzugte Werte aufweisen.

So ist in einer bevorzugten Ausführungsform der erfindungsgemäße Katalysator dadurch gekennzeichnet, dass die im 2θ (2 Theta) Bereich von 5° bis 80° aufgenommene XRD Diffraktogramm mindestens die Beugungsreflexen bei 26,54° 2θ (2 Theta), 28,12° 2θ (2 Theta), 29,90° 2θ (2 Theta), 34,55° 2θ (2 Theta), 49,70° 2θ (2 Theta), 53,90° 2θ (2 Theta), 59,12° 2θ (2 Theta), 61,96° 2θ (2 Theta), 66,42° 2θ (2 Theta), 73,48°2θ (2 Theta) aufweist.

Die XRD-Analysen wurden mit einem D8 Advance Serie 2 von der Firma Bruker/AXS unter Verwendung von CuK-alpha-Quelle (mit einer Wellenlänge von 0,154 nm bei 40 kV und 40 mA) und θ-θ Geometrie (Bragg-Brentano Geometrie) in Reflexionsmodus durchgeführt. Die Messungen erfolgten über den Messbereich: 5-80° (2 Theta), 0.02° Schritten mit 3.6 Sekunden/Schritt.

In einer weiter bevorzugten Ausführungsform ist der erfindungsgemäße Katalysator dadurch gekennzeichnet, dass seine BET-Oberfläche bei mindestens 10 m²/g, vorteilhafterweise bei mindestens 20 m²/g, bevorzugt bei mindestens 40 m²/g, besonders bevorzugt bei mindestens 60m²/g und ganz besonders bevorzugt bei mindestens 80m²/g, liegt. Die BET Oberfläche wurde nach der DIN 66131 bestimmt.

In einer weiter bevorzugten Ausführungsform ist der erfindungsgemäße Katalysator dadurch gekennzeichnet, dass sein Porenvolumen vorteilhafterweise im Bereich von 0,05 bis 1,5 cm³/g, bevorzugt im Bereich von 0,1 bis 1,0 cm³/g, besonders bevorzugt im Bereich von 0,15 bis 0,9 cm³/g, ganz besonders bevorzugt im Bereich von 0,17 bis 0,7 cm³/g, insbesondere im Bereich von 0,2 bis 0,6 cm³/g, liegt. Zur Bestimmung des Porenvolumens wurde die Methode der Quecksilber-Porosimetrie nach DIN Norm 66133 herangezogen.

In einer bevorzugten Ausführungsform liegt der erfindungsgemäße Katalysator als Formkörper (wie z.B. Tabletten, Extrudate, kugelförmige Partikel, etc.) vor, wobei die Druckfestigkeit der Formkörper vorteilhafterweise bei mindestens 0,2 kgf, bevorzugt bei mindestens 0,5 kgf, besonders bevorzugt bei mindestens 1,0 kgf, ganz besonders bevorzugt bei mindestens 1,5 kgf, insbesondere bei mindestens 2,0 kgf. Die Seitendruckfestigkeit ist ein Maß für die Stabilität eines Materials bei Ausübung von Druck auf seine Seitenflächen. Das Material wird dazu zwischen zwei Stempeln eingespannt (Vorkraft 0,5 N), die sich dann mit 1,6 mm/min Prüfgeschwindigkeit aufeinander zu bewegen und das Material zerdrücken. Die Kraft, die zum Zerdrücken des Materials benötigt wird, wird aufgezeichnet. Daten ergeben sich über eine statistische Auswertung von wenigstens 20 Formkörpern.

Weitere Ausführungsformen in der Zusammensetzung des erfindungsgemäß eingesetzten Katalysators sind den Beispielen zu entnehmen. Es versteht sich auch hierbei, dass die vorstehend genannten und nachstehend noch angegebenen Merkmale des Katalysators nicht nur in den angegebenen Kombinationen und Wertebereichen, sondern auch in anderen Kombinationen und Wertebereichen in den Grenzen des Hauptanspruchs verwendbar sind, ohne den Rahmen der Erfindung zu verlassen.

Die Herstellung des erfindungsgemäß eingesetzten Katalysators erfolgt auf übliche Art und Weise, beispielsweise indem die Aktivkomponente und ggf. das Dotierelement, vorzugsweise in Form ihrer Salze/Hydrate, in Lösung gebracht und dann in geeigneter Weise, beispielsweise durch Tränken, auf den Träger aufgebracht werden. Danach wird der Katalysator getrocknet, kalziniert, ggf. reduziert und ggf. passiviert.

Das Aufbringen der Aktivkomponenten durch Tränken auf das Trägermaterial kann auf übliche Art und Weise erfolgen, wie z. B. als Washcoat auf einen Monolithen. Durchführung und Verfahrensbedingungen sind beispielsweise im Handbook of heterogeneous catalysis, 2nd edition, Vol. 1, VCH Verlagsgesellschaft Weinheim, 2008, Seiten 57 bis 66 und 147 bis 149, beschrieben.

Eine alternative Herstellungsweise beinhaltet die Verknetung der Trägermaterialien mit den Salzen/Hydraten der Aktiv- und ggf. Dotierelemente mit anschließender Verstrangung, Trocknung und ggf. Kalzination, ggf. Reduktion und ggf. Passivierung.

Dabei können das Verkneten des Trägermaterials mit den Aktivmassen sowie die weiteren Arbeitsschritte auf übliche Art und Weise mit bekannten Apparaturen erfolgen.

Die Herstellung von Formkörpern aus pulverförmigen Rohstoffen kann durch übliche, dem Fachmann bekannte Methoden, wie beispielsweise Tablettierung, Aggregation oder Extrusion erfolgen, wie sie u.a. im Handbook of Heterogeneous Catalysis, Vol. 1, VCH Verlagsgesellschaft Weinheim, 1997, Seiten 414-417 beschrieben sind, erfolgen. Bei der Verformung bzw. dem Aufbringen können dem Fachmann bekannte Hilfsstoffe, wie Binder, Schmiermittel und/oder Lösungsmittel zugesetzt werden.

Es entsteht eine katalytisch aktive Zusammensetzung, die für die selektive Methanisierung von Kohlenmonoxid in wasserstoff- und kohlendioxidhaltigen Reformatströmen hervorragend geeignet ist. In Abhängigkeit von den jeweiligen Reaktionsbedingungen wird dabei die gewünschte deutliche Abreicherung des CO kleiner 10ppm im Gasgemisch unter minimalem Verlust an Wasserstoff erreicht.

Gegenstand der Erfindung ist auch die Verwendung einer katalytisch aktiven Zusammensetzung für die selektive Methanisierung von Kohlenmonoxid in wasserstoff- und kohlendioxidhaltigen Reformatströmen, die dadurch gekennzeichnet ist, dass sie als Aktivkomponente wenigstens ein Element, ausgewählt aus der Gruppe, bestehend aus Ruthenium, Rhodium, Nickel und Kobalt, bevorzugt Ruthenium und als Dotierungsmittel Rhenium auf einem Trägermaterial, ausgewählt aus der Gruppe, bestehend aus Al₂O₃, ZrO₂, TiO₂, SiC, ZnO, Oxiden der Gruppe IIA Metalle, Oxiden der Gruppen IIIB, IVB, VB, VIB Übergangsmetalle, Oxiden von Metallen der Seltenen Erden, Aluminosilicate, Zeolite, MOFs (Metal Organic Framework) sowie deren Mischungen, enthält.

Eine bevorzugte Ausführungsform der erfindungsgemäßen Verwendung der katalytisch aktiven Zusammensetzung für die selektive Methanisierung von Kohlenmonoxid in wasserstoff- und kohlendioxidhaltigen Reformatströmen ist dadurch gekennzeichnet, dass eine katalytisch aktive Zusammensetzung eingesetzt wird, die als Aktivkomponente wenigstens ein Element, ausgewählt aus der Gruppe, bestehend aus Ruthenium, Rhodium, Nickel und Kobalt, bevorzugt Ruthenium und als Dotierungsmittel Rhenium auf einem Trägermaterial enthält, wobei die Gesamtbeladung des Trägermaterials mit der Aktivkomponente 0,1 bis 20 Gew.-% und mit Rhenium 0,01 bis 20 Gew.-%, jeweils bezogen auf das Gesamtgewicht der katalytisch aktiven Zusammensetzung beträgt, und das Trägermaterial eine oder mehrere Komponenten, ausgewählt aus der Gruppe, bestehend aus Al₂O₃, ZrO₂, TiO₂, SiC, ZnO, Oxide der Gruppe IIA Metalle, Oxide der Gruppen IIIB, IVB, VB, VIB Übergangsmetalle, Oxiden von Metallen der Seltenen Erden, Aluminosilicate, Zeolite, MOFs (Metal Organic Framework) sowie deren Mischungen enthält.

Eine besonders bevorzugte Ausführungsform der erfindungsgemäßen Verwendung der katalytisch aktiven Zusammensetzung für die selektive Methanisierung von Kohlenmonoxid in wasserstoff- und kohlendioxidhaltigen Reformatströmen ist dadurch gekennzeichnet, dass eine katalytisch aktive Zusammensetzung eingesetzt wird, die als Aktivkomponente wenigstens ein Element, ausgewählt aus der Gruppe, bestehend aus Ruthenium, Rhodium, Nickel und Kobalt, bevorzugt Ruthenium und als Dotierungsmittel Rhenium auf einem Trägermaterial enthält, wobei die Gesamtbeladung des Trägermaterials mit der Aktivkomponente 0,1 bis 20 Gew.-%, bevorzugt 0,2 bis 10 Gew.-%, besonders bevorzugt 0,3 bis 5 Gew.-% und mit Rhenium 0,01 bis 20 Gew.-%, bevorzugt 0,07 bis 5 Gew.-%, besonders bevorzugt 0,08 bis 4 Gew.-%, jeweils bezogen auf das Gesamtgewicht der katalytisch aktiven Zusammensetzung beträgt, und das Trägermaterial bevorzugt eine Komponente, ausgewählt aus der Gruppe, bestehend aus Zeolit A, ß-Zeolit, Mordenit, Faujasit, ZSM-5 und MOF, enthält.

Eine weitere besonders bevorzugte Ausführungsform der erfindungsgemäßen Verwendung der katalytisch aktiven Zusammensetzung für die selektive Methanisierung von Kohlenmonoxid in wasserstoff- und kohlendioxidhaltigen Reformatströmen ist dadurch gekennzeichnet, dass eine katalytisch aktive Zusammensetzung eingesetzt wird, die als Aktivkomponente wenigstens ein Element, ausgewählt aus der Gruppe, bestehend aus Ruthenium, Rhodium, Nickel und Kobalt, bevorzugt Ruthenium und als Dotierungsmittel Rhenium auf einem Trägermaterial enthält, wobei die Gesamtbeladung des Trägermaterials mit der Aktivkomponente 0,1 bis 20 Gew.-%, bevorzugt 0,2 bis 10 Gew.-%, besonders bevorzugt 0,3 bis 5 Gew.-% und mit Rhenium 0,01 bis 20 Gew.-%, bevorzugt 0,07 bis 5 Gew.-%, besonders bevorzugt 0,08 bis 4 Gew.-%, jeweils bezogen auf das Gesamtgewicht der katalytisch aktiven Zusammensetzung beträgt, und das Trägermaterial bevorzugt ein Lanthan-Cer-Zirkonoxid enthält, wobei das Trägermaterial einen Lanthanoxid-Gehalt von 0,1 bis 15 Gew.-%, einen Ceroxid-Gehalt von 0,1 bis 20 Gew.-% und einen Zirkonoxid-Gehalt von 30 bis 99,8 Gew.-%, bezogen auf das Gewicht des gesamten Trägermaterials, enthält.

Gegenstand der Erfindung ist ebenfalls ein Verfahren zur selektiven Methanisierung von Kohlenmonoxid in wasserstoff- und kohlendioxidhaltigen Reformatströmen, dadurch gekennzeichnet, dass eine katalytisch aktive Zusammensetzung eingesetzt wird, die als Aktivkomponente wenigstens ein Element, ausgewählt aus der Gruppe, bestehend aus Ruthenium, Rhodium, Nickel und Kobalt, bevorzugt Ruthenium und als Dotierungsmittel Rhenium auf einem Trägermaterial, ausgewählt aus der Gruppe, bestehend aus Al₂O₃, ZrO₂, TiO₂, SiC, ZnO, Oxiden der Gruppe IIA Metalle, Oxiden der Gruppen IIIB, IVB, VB, VIB Übergangsmetalle, Oxiden von Metallen der Seltenen Erden, Aluminosilicate, Zeolite, MOFs (Metal Organic Framework) sowie deren Mischungen, enthält.

Eine bevorzugte Ausführungsform des erfindungsgemäßen Verfahrens zur selektiven Methanisierung von Kohlenmonoxid in wasserstoff- und kohlendioxidhaltigen Reformatströmen ist dadurch gekennzeichnet, dass eine katalytisch aktive Zusammensetzung eingesetzt wird, die als Aktivkomponente wenigstens ein Element, ausgewählt aus der Gruppe, bestehend aus Ruthenium, Rhodium, Nickel und Kobalt, bevorzugt Ruthenium und als Dotierungsmittel Rhenium auf einem Trägermaterial enthält, wobei die Gesamtbeladung des Trägermaterials mit der Aktivkomponente 0,1 bis 20 Gew.-%, und mit Rhenium 0,01 bis 20 Gew.-%, jeweils bezogen auf das Gesamtgewicht der katalytisch aktiven Zusammensetzung beträgt, und das Trägermaterial eine oder merhere Komponenten, ausgewählt aus der Gruppe, bestehend aus Al₂O₃, ZrO₂, TiO₂, SiC, ZnO, Oxide der Gruppe IIA Metalle, Oxide der Gruppen IIIB, IVB, VB, VIB Übergangsmetalle, Oxiden von Metallen der Seltenen Erden, Aluminosilicate, Zeolite, MOFs (Metal Organic Framework) sowie deren Mischungen enthält.

Eine besonders bevorzugte Ausführungsform des erfindungsgemäßen Verfahrens zur selektiven Methanisierung von Kohlenmonoxid in wasserstoff- und kohlendioxidhaltigen Reformatströmen ist dadurch gekennzeichnet, dass eine katalytisch aktive Zusammensetzung eingesetzt wird, die als Aktivkomponente wenigstens ein Element, ausgewählt aus der Gruppe, bestehend aus Ruthenium, Rhodium, Nickel und Kobalt, bevorzugt Ruthenium und als Dotierungsmittel Rhenium auf einem Trägermaterial enthält, wobei die Gesamtbeladung des Trägermaterials mit der Aktivkomponente 0,1 bis 20 Gew.-%, bevorzugt 0,2 bis 10 Gew.-%, besonders bevorzugt 0,3 bis 5 Gew.-% und mit Rhenium 0,01 bis 20 Gew.-%, bevorzugt 0,07 bis 5 Gew.-%, besonders bevorzugt 0,08 bis 4 Gew.-%, jeweils bezogen auf das Gesamtgewicht der katalytisch aktiven Zusammensetzung beträgt, und das Trägermaterial bevorzugt eine Komponente, ausgewählt aus der Gruppe, bestehend aus Zeolit A, ß-Zeolit, Mordenit, Faujasit, ZSM-5 und MOF, enthält.

Eine weitere besonders bevorzugte Ausführungsform des erfindungsgemäßen Verfahrens zur selektiven Methanisierung von Kohlenmonoxid in wasserstoff- und kohlendioxidhaltigen Reformatströmen ist dadurch gekennzeichnet, dass eine katalytisch aktive Zusammensetzung eingesetzt wird, die als Aktivkomponente wenigstens ein Element ausgewählt aus der Gruppe bestehend aus Ruthenium, Rhodium, Nickel und Kobalt, bevorzugt Ruthenium und als Dotierungsmittel Rhenium auf einem Trägermaterial enthält, wobei die Gesamtbeladung des Trägermaterials mit der Aktivkomponente 0,1 bis 20 Gew.-%, bevorzugt 0,2 bis 10 Gew.-%, besonders bevorzugt 0,3 bis 5 Gew.-% und mit Rhenium 0,01 bis 20 Gew.-%, bevorzugt 0,07 bis 5 Gew.-%, besonders bevorzugt 0,08 bis 4 Gew.-%, jeweils bezogen auf das Gesamtgewicht der katalytisch aktiven Zusammensetzung beträgt, und das Trägermaterial bevorzugt ein Lanthan-Cer-Zirkonoxid enthält, wobei das Trägermaterial einen Lanthanoxid-Gehalt von 0,1 bis 15 Gew.-%, einen Ceroxid-Gehalt von 0,1 bis 20 Gew.-% und einen Zirkonoxid-Gehalt von 30 bis 99,8 Gew.-%, bezogen auf das Gewicht des gesamten Trägermaterials, enthält.

Das erfindungsgemäße selektive Methanisierungsverfahren kann in einem Temperaturbereich von vorzugsweise 100 bis 300°C ausgeübt werden.

Besonders vorteilhaft ist die selektive Methanisierung von CO in einem Temperaturbereich von 180 bis 260°C. Diese Temperatur ermöglicht eine direkte thermische Integration an die vorgeschaltete Tieftemperaturkonvertierung. Es wird damit möglich, die erfindungsgemäße Methanisierungsstufe unmittelbar an die Tieftemperaturkonvertierungsstufe anzukoppeln. Durch die hohe Aktivität bei gleichermaßen hoher CO-Selektivität in diesem Temperaturbereich wird gewährleistet, dass ein stabiler und vor allem thermisch integrierter Betrieb des Katalysators erst möglich wird.

Das erfindungsgemäße Verfahren wird bei einer Fahrweise betrieben, deren GHSV in einem Bereich von 200 bis 20000 h⁻¹, bevorzugt in einem Bereich von 500 bis 15000 h⁻¹, besonders bevorzugt in einem Bereich von 1000 bis 10000 h⁻¹, und ganz besonders bevorzugt in einem Bereich von 2000 bis 7500 h⁻¹ liegt. Die GHSV "Gas hourly space velocity" ist eine Angabe über den Gasfluss eines Reaktionsgases in Litern pro Liter Katalysator und pro Stunde bei Standardtemperatur und Standarddruck.

Das erfindungsgemäße Verfahren zur selektiven Methanisierung von Kohlenmonoxid in wasserstoff- und kohlendioxidhaltigen Strömen an dem erfindungsgemäßen hochaktiven Methanisierungskatalysator erfolgt in üblichen Apparaturen und unter üblichen Bedingungen zur Durchführung einer Methanisierungsreaktion, wie sie beispielsweise im Handbook of heterogeneous catalysis, 2nd edition, Vol. 1, VCH Verlagsgesellschaft Weinheim, 2008, Seite 353, beschrieben sind, und unter Überströmen des Katalysators mit einem CO und Wasserstoffhaltigen Prozessgas.

Das für das Methanisierungsverfahren geeignete Prozessgas ist ein Synthesegas, das durch Reformierung von festen, flüssigen und gasförmigen Brennstoffen erzeugt werden kann. Als bevorzugte Brennstoffe sind Erdgas, Flüssiggas (LPG), langkettige Kohlenwasserstoffe (Benzin, Diesel), sowie Alkohole wie Methanol oder Ethanol zu nennen. Unter Reformierung sind die für den Fachmann bekannten Prozesse wie die Dampfreformierung, partielle Oxidation sowie die autotherme Reformierung zu verstehen. Als bevorzugte Reformierungsverfahren sind die Dampfreformierung und autotherme Reformierung von Kohlenwasserstoffen wie Erdgas, Benzin und Diesel gemeint.

Die katalytisch aktive Zusammensetzung eignet sich damit hervorragend für CO-Feinreinigungen in wasserstoff- und kohlendioxidhaltigen Reformatströmen, insbesondere für den Einsatz bei der Wasserstofferzeugung für Brennstoffzellenanwendungen.

Die Erfindung wird anhand der nachfolgenden Ausführungsbeispiele näher erläutert, ohne jedoch hierdurch eine entsprechende Eingrenzung vorzunehmen.

### Beispiele

### Beispiel 1:

148,1 g eines Lanthan-Cer-Zirkonoxid-Trägers (enthaltend 65 Gew.-% ZrO₂, 15 Gew.-% CeO₂, 5 Gew.-% La₂O₃ und 15 Gew.-% Al₂O₃) wurden mit einer ca. 30 Gew.-%igen RuCl₃-Lösung imprägniert, deren Menge so eingestellt war, dass der fertige Katalysator 2 Gew.-% Ru als Aktivmasse trug. Anschließend wurde der imprägnierte Träger in einem Drehrohrofen bei 120°C sechzehn Stunden getrocknet und danach für zwei Stunden bei 475°C kalziniert (mit einer Aufheizrate von 4°C/min). Der so erhaltene Ruthenium Katalysator wurde anschließend mit einer Perrheniumsäure-Lösung (HReO₄) imprägniert und erneut für sechzehn Stunden bei 120°C getrocknet. Die Konzentration an Perrheniumsäure wurde so eingestellt, dass der fertige Katalysator nach der Trocknung 2 Gew.-% Re als Dotiermittel enthielt. Die BET-Oberfläche des fertigen Katalysators betrug 83 m²/g*⁾.

### Beispiel 2

148,1 g eines Lanthan-Cer-Zirkonoxid-Trägers (enthaltend 65 Gew.-% ZrO₂, 15 Gew.-% CeO₂, 5 Gew.-% La₂O₃ und 15 Gew.-% Al₂O₃) wurden mit einer ca. 30 Gew.-%igen RuCl₃-Lösung imprägniert, deren Menge so eingestellt war, dass der fertige Katalysator 1 Gew.-% Ru als Aktivmasse trug. Anschließend wurde der imprägnierte Träger in einem Drehrohrofen bei 120°C sechzehn Stunden getrocknet und danach für zwei Stunden bei 475°C kalziniert (mit einer Aufheizrate von 4°C/min). Der so erhaltene Ruthenium Katalysator wurde anschließend mit einer Perrheniumsäure-Lösung (HReO₄) imprägniert und erneut für sechzehn Stunden bei 120°C getrocknet. Die Konzentration an Perrheniumsäure wurde so eingestellt, dass der fertige Katalysator nach der Trocknung 1 Gew.-% Re als Dotiermittel enthielt. Die BET-Oberfläche des fertigen Katalysators betrug 86 m²/g*⁾.

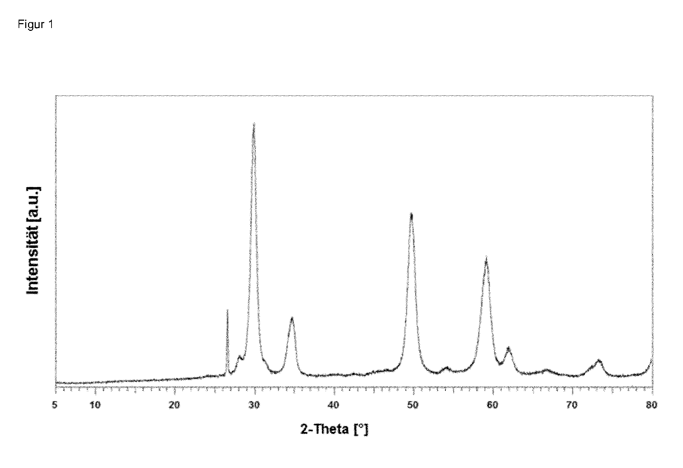

Die XRD Diffraktorgramm dieses Katalysators ist in Figur 1 dargestellt.

### Beispiel 3

148,1 g eines Lanthan-Cer-Zirkonoxid-Trägers (enthaltend 65 Gew.-% ZrO₂, 15 Gew.-% CeO₂, 5 Gew.-% La₂O₃ und 15 Gew.-% Al₂O₃) wurden mit einer ca. 30 Gew.-%igen RuCl₃-Lösung imprägniert, deren Menge so eingestellt war, dass der fertige Katalysator 2 Gew.-% Ru als Aktivmasse trug. Anschließend wurde der imprägnierte Träger in einem Drehrohrofen bei 120°C sechzehn Stunden getrocknet und danach für zwei Stunden bei 475°C kalziniert (mit einer Aufheizrate von 4°C/min). Der so erhaltene Ruthenium Katalysator wurde anschließend mit einer Perrheniumsäure-Lösung (HReO₄) imprägniert und erneut für sechzehn Stunden bei 120°C getrocknet. Die Konzentration an Perrheniumsäure wurde so eingestellt, dass der fertige Katalysator nach der Trocknung 0,5 Gew.-% Re als Dotiermittel enthielt. Die BET-Oberfläche des fertigen Katalysators betrug 85 m²/g*⁾.

### Beispiel 4

148,1 g eines Lanthan-Cer-Zirkonoxid-Trägers (enthaltend 65 Gew.-% ZrO₂, 15 Gew.-% CeO₂, 5 Gew.-% La₂O₃ und 15 Gew.-% Al₂O₃) wurden mit einer ca. 30 Gew.-%igen RuCl₃-Lösung imprägniert, deren Menge so eingestellt war, dass der fertige Katalysator 2 Gew.-% Ru als Aktivmasse trug. Anschließend wurde der imprägnierte Träger in einem Drehrohrofen bei 120°C sechzehn Stunden getrocknet und danach für zwei Stunden bei 475°C kalziniert (mit einer Aufheizrate von 4°C/min). Der so erhaltene Ruthenium Katalysator wurde anschließend mit einer Perrheniumsäure-Lösung (HReO₄) imprägniert und erneut für sechzehn Stunden bei 120°C getrocknet. Die Konzentration an Perrheniumsäure wurde so eingestellt, dass der fertige Katalysator nach der Trocknung 0,25 Gew.-% Re als Dotiermittel enthielt. Die BET-Oberfläche des fertigen Katalysators betrug 88 m²/g*⁾.

### Beispiel 5

148,1 g eines Lanthan-Cer-Zirkonoxid-Trägers (enthaltend 65 Gew.-% ZrO₂, 15 Gew.-% CeO₂, 5 Gew.-% La₂O₃ und 15 Gew.-% Al₂O₃) wurden mit einer ca. 30 Gew.-%igen RuCl₃-Lösung imprägniert, deren Menge so eingestellt war, dass der fertige Katalysator 2 Gew.-% Ru als Aktivmasse trug. Anschließend wurde der imprägnierte Träger in einem Drehrohrofen bei 120°C sechzehn Stunden getrocknet und danach für zwei Stunden bei 475°C kalziniert (mit einer Aufheizrate von 4°C/min). Der so erhaltene Ruthenium Katalysator wurde anschließend mit einer Perrheniumsäure-Lösung (HReO₄) imprägniert und erneut für sechzehn Stunden bei 120°C getrocknet. Die Konzentration an Perrheniumsäure wurde so eingestellt, dass der fertige Katalysator nach der Trocknung 0,1 Gew.-% Re als Dotiermittel enthielt. Die BET-Oberfläche des fertigen Katalysators betrug 86 m²/g*⁾.
*⁾ Die BET Oberfläche der jeweiligen erfindungsgemäßen Katalysatoren wurde nach der DIN 66131 bestimmt.

### Beispiel 6 (Vergleichsbeispiel)

(Patentnachstellung EP 2 125 201 B1, Beispiel 7d)

Ein Träger aus 70 Gew.-% ZrO₂, 15 Gew.-% CeO₂, 5 Gew.-% La₂O₃ und 10 Gew.-% Al₂O₃ wurde mit einer RuCl₃-Lösung versetzt, deren Konzentration so eingestellt war, dass das kalzinierte Endprodukt 2 Gew.-% Ru als Aktivmasse trug.

### Beispiel 7 (Vergleichsbeispiel)

147 g eines γ-Al₂O₃-Trägers (0,8 mm Kugeln, Sasol GmbH) wurden mit einer ca. 30 Gew.-%igen RuCl₃-Lösung imprägniert, deren Menge so eingestellt war, dass der fertige Katalysator 2 Gew.-% Ru als Aktivmasse trug. Anschließend wurde der imprägnierte Träger in einem Drehrohrofen bei 120°C sechzehn Stunden getrocknet und danach für zwei Stunden bei 475°C kalziniert (mit einer Aufheizrate von 4°C/min).

**Tabelle 1:**

| Zusammensetzung der Katalysatoren der Beispiele 1 bis 7 | | | |
|---|---|---|---|
| Katalysator | Ru [Gew.-%] | Re [Gew.-%] | Träger |
| Beispiel 1 | 2 | 2 | LaCeZr-Oxid |
| Beispiel 2 | 2 | 1 | LaCeZr-Oxid |
| Beispiel 3 | 2 | 0,5 | LaCeZr-Oxid |
| Beispiel 4 | 2 | 0,25 | LaCeZr-Oxid |
| Beispiel 5 | 2 | 0,10 | LaCeZr-Oxid |
| Beispiel 6* | 2 | 0 | LaCeZr-Oxid |
| Beispiel 7* | 2 | 0 | Al₂O₃ |

| | | | |
|---|---|---|---|
| * Vergleichsbeispiele | | | |

### Beispiel 8 - Selektive Methanisierung unter Verwendung der Katalysatoren aus den Beispielen 1 bis 7

### Testbedingungen:

Für den Versuch wurde ein elektrisch beheizter Festbett-Rohrreaktor mit einer Länge von 530 mm und einem inneren Durchmesser von 10 mm verwendet.

Zuunterst wurden 5 ml Steatit-Kugeln mit einem Durchmesser von 1,8 bis 2,2 mm eingebaut, auf die anschließend die Katalysatormischung gegeben wurde. Die Katalysatormischung bestand aus ca. 20 ml Katalysator-Pellets (1,5 x 1,5 mm). Als Vorschüttung dienten 5 ml Steatit-Kugeln mit einem Durchmesser von 1,8 bis 2,2 mm, die das Restvolumen des Reaktors füllten.

Der Katalysator wurde zunächst mit 90 l/h Stickstoff und 10 l/h Wasserstoff bei 230°C eine Stunde lang reduziert. Die für den Versuch gewählte Gaszusammensetzung ist typisch für den Ausgang der Tieftemperatur-Shiftstufe nach der Reformierung von Methan und betrug 22 Vol.-% H₂, 28 Vol.-% N₂, 25 Vol.-% H₂O, 13 Vol.-% CO₂, 5 Vol.-% CO und 0,5 Vol.-% CH₄. Alle Versuche wurden bei einem Druck von 2 bara und einer Belastung von 5000 l·h⁻¹·l⁻¹_{Kat} durchgeführt.

Nachdem alle Gase eingestellt und der Reaktor (nach der Reduktion bei 230°C) auf eine Temperatur von 260°C aufgeheizt worden war, wurde der Versuch gestartet und die Selektivität der jeweils eingesetzten Katalysatoren über einen Zeitraum von 90 Stunden verfolgt.

Die Konzentration der Gase wurde mittels online GC hinter dem Reaktor bestimmt.

Zur Auswertung der Ergebnisse der Beispiele wurden die Größen Selektivität und Umsatz herangezogen. Die Selektivität ist der Quotient von umgesetzter Menge CO und der Menge an entstandenem Methan (in Vol.-%). Der Umsatz bezieht sich auf CO.

### Ergebnisse:

Unter den oben genannten Bedingungen wurden die Katalysatoren vermessen. Mit allen Katalysatoren aus den Beispielen 1 bis 7 konnte unter diesen Versuchsbedingungen ein vollständiger Umsatz von CO erzielt werden (CO-Gehalt = 0 ppm, bzw. unter Detektionsminimum vom GC Gerät).

Die CO-Selektivitäten zu Beginn des jeweiligen Versuchs [Start of Run (SOR)] sowie nach 90 Stunden Versuchsdauer [Time on Stream (TOS)] sind in Tabelle 2 aufgeführt.

Wie aus der Tabelle 2 zu entnehmen ist, fällt die CO-Selektivität bei Verwendung der Vergleichskatalysatoren aus den Beispielen 6 und 7 nach 90 stündiger Betriebsdauer deutlich ab auf Werte von 18 und 24%, während im Falle der erfindungsgemäßen Katalysatoren aus den Beispielen 1 bis 5 noch eine CO-Selektivität zwischen 46% und 53% zu beobachten war.

**Tabelle 2:**

| Ergebnisse der selektiven Methanisierung von CO | | | |
|---|---|---|---|
| | | Selektivität bei 260°C | |
| Katalysator | Aktivmasse/Dotierelement Träger | Start of Run | Nach 90 Stunden TOS |
| Beispiel 1: | 2 Gew.-% Ru/ 2 Gew.% Re LaCeZr-Oxid | 83% | 51% |
| Beispiel 2: | 2 Gew.-% Ru/ 1 Gew.% Re LaCeZr-Oxid | 84% | 53% |
| Beispiel 3: | 2 Gew.-% Ru/ 0,5 Gew.% Re LaCeZr-Oxid | 82% | 49% |
| Beispiel 4: | 2 Gew.-% Ru/ 0,25 Gew.% Re LaCeZr-Oxid | 80% | 45% |
| Beispiel 5: | 2 Gew.-% Ru/ 0,1 Gew.% Re LaCeZr-Oxid | 82% | 46% |
| Beispiel 6: | 2% Ru/ - LaCeZr-Oxid | 81% | 24% |
| Beispiel 7: | 2% Ru/ - γ-Al₂O₃ | 80% | 18% |

**Tabelle 3:**

| Selektivitätsprofil nach 90 Stunden Betriebsdauer bei 260°C und anschließender stufenweiser Temperaturerniedrigung um jeweils 20°C innerhalb von 4 Stunden. Die angegebenen Selektivitätswerte wurden bestimmt bei vollständigem Umsatz von CO (0ppm CO). Im Falle eines nicht vollständigen Umsatzes galt für die Selektivität: = not applicable (n/a). (Test Bedingungen: T=200-260°C, p=2bar, GHSV=5000h⁻¹, Eingangs-Gaszusammensetzung: 5% CO, 13% CO₂, 0,5% CH₄, 22% H₂, 25% H₂O, 28% N₂) | | | | | |
|---|---|---|---|---|---|
| Katalysator | Aktivmasse/Dotierelement Träger | Selektivitätsprofil nach90 h bei verschiedenen Tempeperaturen | | | |
| | | 260°C | 240°C | 220°C | 200°C |
| Beispiel 1 | 2 Gew-% Ru/2 Gew-% Re LaCeZr-Oxid | 49% | 67% | 87% | n/a |
| Beispiel 2 | 2 Gew-% Ru/1 Gew-% Re LaCeZr-Oxid | 50% | 62% | 81% | 100% |
| Beispiel 3 | 2 Gew-% Ru/0,5 Gew-% Re LaCeZr-Oxid | 49% | 66% | 83% | 97% |
| Beispiel 4 | 2 Gew-% Ru/0,25 Gew-% Re LaCeZr-Oxid | 45% | 61% | 79% | 95% |
| Beispiel 5 | 2 Gew-% Ru/0,1 Gew-% Re LaCeZr-Oxid | 46% | 58% | 77% | 95% |
| Beispiel 6 | 2%Ru LaCeZr-Oxid | 24% | 36% | 57% | 88% |
| Beispiel 7 | 2%Ru γ-Al₂O₃ | 18% | 32% | 54% | 95% |

Wie aus Tabelle 3 zu erkennen ist, zeigen die erfindungsgemäßen Rhenium dotierten Rutheniumkatalysatoren aus den Beispielen 1 bis 5 signifikant höhere CO-Selektivitäten über den Temperaturbereich von 200 bis 260°C als die beiden Rheniumfreien Katalysatoren aus den Vergleichsbeispielen 6 und 7.

## Patentansprüche

1. Katalytisch aktive Zusammensetzung für die selektive Methanisierung von Kohlenmonoxid in wasserstoff- und kohlendioxidhaltigen Reformatströmen, enthaltend als Aktivkomponente wenigstens ein Element, ausgewählt aus der Gruppe, bestehend aus Ruthenium, Rhodium, Nickel und Kobalt und als Dotierungsmittel Rhenium auf einem Trägermaterial.

2. Katalytisch aktive Zusammensetzung nach Anspruch 1, **dadurch gekennzeichnet, dass** die Aktivkomponente in einer Menge von 0,1 bis 20 Gew.-% und Rhenium in einer Menge von 0,01 bis 20 Gew.-%, jeweils bezogen auf die Gesamtmenge der katalytisch aktiven Zusammensetzung, vorliegt.

3. Katalytisch aktive Zusammensetzung nach Anspruch 1 oder 2, **dadurch gekennzeichnet, dass** sie als Aktivkomponente Ruthenium enthält.

4. Katalytisch aktive Zusammensetzung nach einem der Ansprüche 1 bis 3, **dadurch gekennzeichnet, dass** sie als Trägermaterial ein Lanthan-Cer-Zirkonoxid enthält.

5. Katalytisch aktive Zusammensetzung nach Anspruch 4, **dadurch gekennzeichnet, dass** das Trägermaterial Lanthanoxid in einer Menge von 0,1 bis 15 Gew.-%, Ceroxid in einer Menge von 0,1 bis 20 Gew.-% und Zirkonoxid in einer Menge von 30 bis 99,8 Gew.-%, jeweils bezogen auf die Gesamtmenge des Trägermaterials, enthält.

6. Verfahren zur Herstellung einer katalytisch aktiven Zusammensetzung gemäß einem der Ansprüche 1 bis 5, enthaltend die Schritte, dass die Aktivkomponente und das Dotierungsmittel in Lösung gebracht und durch Tränken auf das Trägermaterial aufgebracht werden.

7. Verfahren zur Herstellung einer katalytisch aktiven Zusammensetzung gemäß einem der Ansprüche 1 bis 5, enthaltend die Schritte, dass das Trägermaterial mit den Salzen und/oder Hydraten der Aktivkomponente und des Dotierungsmittels verknetet und anschließend verstrangt und getrocknet wird.

8. Verwendung einer katalytisch aktiven Zusammensetzung gemäß einem der Ansprüche 1 bis 5 für die selektive Methanisierung von Kohlenmonoxid in wasserstoff- und kohlendioxidhaltigen Reformatströmen.

9. Verfahren zur selektiven Methanisierung von Kohlenmonoxid, **dadurch gekennzeichnet, dass** eine katalytisch aktive Zusammensetzung eingesetzt wird, die als Aktivkomponente wenigstens ein Element, ausgewählt aus der Gruppe, bestehend aus Ruthenium, Rhodium, Nickel und Kobalt und als Dotierungsmittel Rhenium auf einem Trägermaterial enthält.

10. Verfahren nach Anspruch 9, **dadurch gekennzeichnet, dass** die Methanisierung in einem Temperaturbereich von 100 bis 300°C erfolgt.

11. Verfahren nach Anspruch 9, **dadurch gekennzeichnet, dass** es einer Tieftemperaturkonvertierungsstufe direkt nachgeschaltet ist.

12. Verwendung einer katalytisch aktiven Zusammensetzung gemäß einem der Ansprüche 1 bis 5 als Verfahrensschritt in der Wasserstofferzeugung für Brennstoffzellenanwendungen.
